(19) **Europäisches Patentamt**
**European Patent Office**
**Office européen des brevets**

(11) **EP 3 600 154 B1**

(12) **EUROPEAN PATENT SPECIFICATION**

(45) Date of publication and mention
of the grant of the patent:
**22.10.2025 Bulletin 2025/43**

(21) Application number: **18774365.3**

(22) Date of filing: **30.03.2018**

(51) International Patent Classification (IPC):
***A61F 2/18*** *(2006.01)*

(52) Cooperative Patent Classification (CPC):
**A61F 2/18;** A61F 2002/183

(86) International application number:
**PCT/PL2018/050014**

(87) International publication number:
**WO 2018/182439 (04.10.2018 Gazette 2018/40)**

(54) **MIDDLE EAR PROSTHESIS**

MITTELOHRPROTHESE

PROTHÈSE D'OREILLE MOYENNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.03.2017 PL 42107517**

(43) Date of publication of application:
**05.02.2020 Bulletin 2020/06**

(73) Proprietors:
• **Uniwersytet Jagiellonski
31-007 Krakow (PL)**
• **Akademia Gorniczo-Hutnicza im. Stanislawa
Staszica w Krakowie
30-059 Krakow (PL)**

(72) Inventors:
• **KONIOR, Marcin
30-316 Kraków (PL)**
• **KLACZYNSKI, Maciej
30-318 Kraków (PL)**

(74) Representative: **Witek, Rafal
WTS Patent Attorneys
Witek, Sniezko & Partners
ul. Weigla 12
53-114 Wroclaw (PL)**

(56) References cited:
US-A- 4 624 672     US-A- 4 624 672
US-A- 4 957 507     US-A1- 2001 027 342
US-A1- 2009 198 334     US-B1- 6 277 148
US-B2- 8 057 542

• ANONYMOUS: "Ti-6Al-4V - Wikipedia", 16 October 2020 (2020-10-16), XP055749816, Retrieved from the Internet <URL:https://en.wikipedia.org/wiki/Ti-6Al-4V> [retrieved on 20201112]
• SHOU-I CHEN ET AL.: "Modeling sound transmission of human middle ear and its clinical applications using finite element analysis", KAOHSIUNG JOURNAL OF MEDICAL SCIENCES, vol. 29, no. 3, March 2013 (2013-03-01), pages 133 - 139, XP055559974, Retrieved from the Internet <URL:https://www.sciencedirect.com/science/article/pii/S1607551X12002240>

**EP 3 600 154 B1**

## Description

The subject of the invention is a middle ear prosthesis, reconstructing the ossicular chain, used in middle ear surgery. The subject of the invention is applied, among others, in the surgical treatment of patients suffering from hearing disorders in the course of middle ear disorders, in particular with conductive hearing loss.

Hearing impairment constitutes the main problem of patients with middle ear disorders. Many of these cases require a surgical intervention. The multitude of prostheses types used in hearing improvement operations indicates the imperfection of the methods developed so far. A restriction of their effectiveness to selected requirements forces individuals performing such procedures to have many expensive models. So far, in the field of middle ear surgery, there have been no solutions, which enable matching one reconstruction system to different intraoperative situations. Therefore, It is necessary to create a prosthesis with wide adaptation possibilities, allowing for effective hearing improvement under various clinical conditions.

In the middle ear pathologies, during surgery, difficulties in reconstruction of the normal auditory pathway may arise. In particular, such a situation involves places where the surfaces of the modelled ossicles are in contact with each other or with the surface previously used to reconstruct prostheses. These are most often zones where prior to the beginning of the disease process articular connections were located, in which a change in the direction of forces and a change in the amount of transmitted acoustic energy took place. Nowadays, an effective and useful ossicular prosthesis, which would be a basis for reconstructing the middle ear conductive chain, allowing for the aid by hearing improvement, which would be possible thanks to surgical treatment in patients suffering from hearing disorders in the course of middle ear disorders, is being sought. Such disorders include mostly chronic otitis media, however, the symptom of conductive hearing loss also occurs in the course of middle ear injuries, congenital defects, tympanosclerosis and middle ear neoplasms. Numerous patients, due to a difficult operating field, which includes the tympanic cavity and middle ear spaces, and because of the imperfections of the currently used ossicular prostheses, require further surgical procedures. Therefore, the search for better and better solutions becomes a necessity and a condition for the development in this field of therapeutic activity.

The Polish patent PL217562B1 discloses a chamber middle ear prosthesis for use in ear surgery to restore the function of the middle ear of a patient. The above-mentioned chamber middle ear prosthesis comprises a conical chamber formed by an annular wall having a shape of a truncated cone side wall whose wider base is closed by a membrane connected to a rigid element transmitting vibrations, and a narrower base is attached to the stapes base. The essence of the solution is that the opening in the narrower base is a capillary opening for filling the conical chamber with a liquid medium, especially a saline solution, and a flat annular disc with elements attaching the prosthesis to the stapes base is joined to the narrower base, the rigid element transmitting vibrations connected to the the membrane being located outside the conical chamber.

In turn, the patent application P.412143 discloses a middle ear prosthesis of variable length. The essence of the middle ear prosthesis of variable length, having a holder fixing the prosthesis to the stapes and an element fixing the prosthesis to the tympanic membrane, is that it consists of S-shaped bars that are attached to the ends in the holder fixing the prosthesis to the stapes and the element fixing the prosthesis to the tympanic membrane. S-shaped bars, the prosthesis is built of, are connected to each other at the point of their contact with the use of stabilizing rings. In addition, the prosthesis is made of a shape memory material.

U.S. Patent No. 6277148B1 discloses, among others, an attachment device for attaching an active middle ear implant to the structures of the middle ear. The attachment device consists of two loops connected with one another at a certain angle, made of wire, one loop being attached to the structures of the middle ear, e.g. to the ossicular chain system, and the other loop being intended to attach and maintain the implant part, e.g. in the form of a magnetic transducer. The above-mentioned attachment device is only an element that fixes the active implant to the structures of the middle ear. It is made of wire of a $TiAl_6V_4$ alloy.

The technical problem addressing the present invention is to provide such a middle ear prosthesis that will effectively replace the ossicular function and thus allow for a reconstruction of the middle ear conductive chain. Furthermore, it is desirable that the middle ear prosthesis effectively transmits sound waves, preferably in the audible range, provides safety of use, provides functionality and intraoperative ease of modelling, and is also a universal structure allowing for the use under a variety of clinical conditions while maintaining a low manufacturing and application cost. Unexpectedly, the above-mentioned technical problems were resolved by the present invention.

The subject of the invention is a middle ear prosthesis as defined in claim 1. In particular it relates to a prosthesis of the middle ear conductive chain, characterized in that it consist of a first spring element constituting a spring of at least one coil, having a length $L_m$ in the range from 0.2 mm to 10 mm, and possibly a second spring element constituting a spring of at least one coil, having a length $L_k$ in the range from 0.1 mm to 4 mm, wherein the first spring element and the second spring element are made of wire of a material having a Young's modulus E in the range from $7 \cdot 10^{10}$ N/m² to $11.4 \cdot 10^{10}$ N/m², a density $\rho$ in the range from $4 \cdot 10^3$ kg/m³ to $20 \cdot 10^3$ kg/m³, a Poisson's ratio v in the range from 0.34 to 0.44, wherein the first spring element is connected to the second spring element in such a way that the rotational symmetry axis of the first spring

2

element is placed at an angle $\alpha_1$ with respect to the rotational symmetry axis of the second spring element, included in the range from 5° to 160°.

In a preferred embodiment of the invention, the middle ear prosthesis consists possibly further of a third spring element attached to the second spring element at the end opposite to the first spring element, wherein the third spring element is a spring of at least one coil, having a length $L_s$ in the range from 0.1 mm to 1 mm, made of wire of a material having a Young's modulus E in the range from $7 \cdot 10^{10}$ N/m$^2$ to $11.4 \cdot 10^{10}$ N/m$^2$, a density $\rho$ in the range from $4 \cdot 10^3$ kg/m$^3$ to $20 \cdot 10^3$ kg/m$^3$, a Poisson's ratio v in the range from 0.34 to 0.44, wherein the third spring element is connected to the second spring element in such a way that the rotational symmetry axis of the third spring element is placed at an angle $\alpha_2$ with respect to the rotational symmetry axis of the second spring element, included in the range from 5° to 160°.

In a further preferred embodiment of the invention, the spring elements form a continuous structure.

In a further preferred embodiment of the invention, the spring elements comprise a compression cylindrical spring, preferably with an outer diameter $D_z$ in the range from 1.0 mm to 1.6 mm.

Preferably, the spring elements are made of wire of a circular cross section having a diameter d in the range from 0.1 mm to 0.3 mm.

Even more preferably, the spring elements have a spring pitch P in the range from 0.1 mm to 1.0 mm.

The middle ear prosthesis of the present invention is characterized by the versatility of use, functionality and intraoperative ease of modelling the final shape. Appropriate rigidity of the prosthesis affects the transfer of acoustic energy with its slight dispersion of it and the possibility of bending the spring, or changing the direction of forces. Proper elasticity allows for the transmission of waves along the axis of the prosthesis, but does not cause excessive energy absorption and the occurrence of a damping effect. The spring construction is characterized by the ability to be easily modelled by bending and twisting, which is extremely important in the spatial structure of the tympanic cavity. The construction of the spring also gives the possibility of easy connection with various anatomical parts of the ossicles to its ends or sides, effectively complementing the conductive chain. It should also be noted that despite the phenomenon of scarring of tissues during the healing process, which normally stiffens the transmission system, the coil structure of the prosthesis can at the same time affect the maintenance of a permanent mobility of the reconstructed system. Thanks to the effective transmission of the sound wave, the middle ear prosthesis of the present invention allows for a replacement of the ossicles function, thereby enabling a reconstruction of the middle ear conductive chain. Thanks to the lack of resonance at low frequencies, the prosthesis does not pose a threat to patients, which indicates the safety of its use. Moreover, the middle ear prosthesis is a simple structure made of a single material, which allows for the achievement of favourable economic factors.

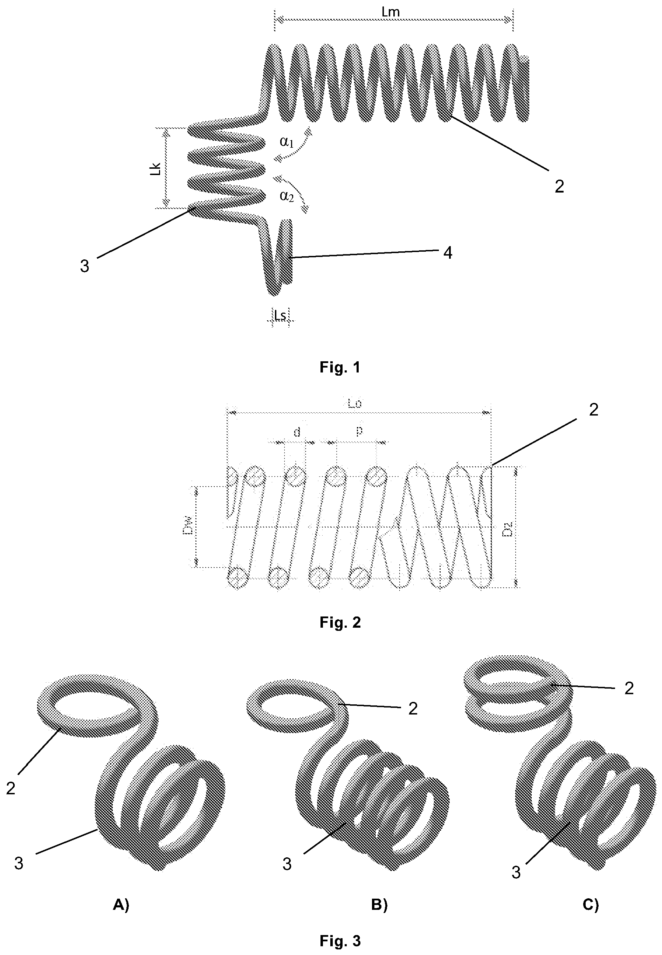

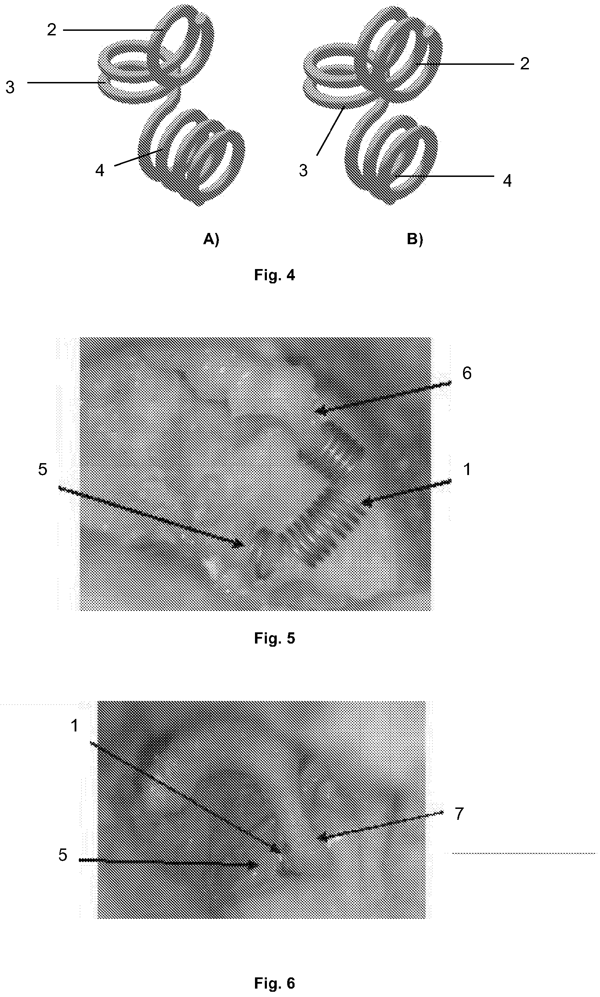

Exemplary embodiments of the invention are presented in the figures of the drawing, in which fig. 1 is a front view of the middle ear prosthesis according to the first embodiment of the invention, fig. 2 is a partial cross-sectional front view of the first spring element of the solution from fig. 1, fig. 3 A-C shows different variants of L-shaped middle ear prostheses in axonometric projection, Figs. 4 A-C shows different variants of C-shaped middle ear prostheses in axonometric projection, fig. 5 is a photograph of an implanted middle ear prosthesis according to one embodiment of the present invention replacing a damaged anvil, whereas fig. 6 is a photograph of an implanted middle ear prosthesis according to one embodiment of the present invention, stabilizing a disengaged incudostapedial joint.

**Example 1**

Fig. 1 is a schematic front view of the middle ear prosthesis 1 according to one possible embodiment of the present invention, while fig. 2 is a partial cross-sectional front view of the first spring element 2 of the solution from fig. 1. The middle ear prosthesis presented in fig. 1 is a structure comprising three segments, made of one wire, constituting a continuous structure. The middle ear prosthesis 1 consists of the first spring element 2, which is a compression cylindrical spring having a length $L_m$ in the range from 0.2 mm to 10 mm (depending on the needs). Fig. 2 indicates important structural parameters of the first spring element 2, also applicable to other spring elements 3, 4 of the discussed middle ear prosthesis 1. The second spring element 2, which is also a compression cylindrical spring having a length $L_k$ in the range from 0.1 mm to 4 mm is connected at one end of the first spring element 2. The first spring element 2 is connected to the second spring element 3 in such a way that the rotational symmetry axis of the first spring element 2 is oriented at an angle $\alpha_1$ with respect to the rotational symmetry axis of the second spring element 3, included in the range from 5° to 160°. Furthermore, the middle ear prosthesis presented in fig. 1 comprises a third spring element 4 attached to the second spring element 3 at the end opposite to the first spring element 2. Similarly to the spring elements 2 and 3, the third spring element 4 is a compression cylindrical spring of at least one coil, having a length $L_s$ in the range from 0.1 mm to 1 mm. Moreover, the third spring element 4 is connected to the second spring element 3 in such a way that the rotational symmetry axis of the third spring element 4 is placed at an angle $\alpha_2$ with respect to the rotational symmetry axis of the second spring element 3, included in the range from 5° to 160°.

The whole middle ear prosthesis, or the spring elements 2, 3, 4 are made of wire of a circular cross section having a diameter d in the range from 0.1 mm to 0.3 mm. Each of the spring elements 2, 3, 4 has an outer diameter $D_z$ in the range from 1.0 to 1.6 mm and a spring pitch P in the range from 0.1 mm to 1.0 mm. The wire used for the construction of the middle ear prosthesis is made of a material having a Young's modulus E in the range from $7 \cdot 10^{10}$ N/m$^2$ to $11.4 \cdot 10^{10}$ N/m$^2$, a density $\rho$ in the range from $4 \cdot 10^3$ kg/m$^3$ to $20 \cdot 10^3$ kg/m$^3$, a Poisson's ratio v in the range from 0.34 to 0.44,

Individual embodiments of the presented middle ear prosthesis 1 will be shown in the following embodiments.

**Example 2**

According to the present invention, ten variants of middle ear prostheses, divided into two groups: L-shaped (variants I - VI) and C-shaped (variants VII - X) have been made. Design parameters of these variants are summarized in Table 1.

Table 1A. Parameters of prostheses most often used in the implantation

| Variant of the prosthesis | Cross section shape | d [mm] | P [mm] | $\alpha_1$ [°] | $\alpha_2$ [°] | $L_m$ [mm] | $L_k$ [mm] | $L_s$ [mm] |
|---|---|---|---|---|---|---|---|---|
| I | "L" | 1.35 | 0.4 | 90 | 0 | 0.4 | 0.8 | - |
| II | "L" | 1.35 | 0.4 | 90 | 0 | 0.4 | 1.6 | - |
| III | "L" | 1.5 | 0.5 | 90 | 0 | 0.5 | 1.0 | - |
| IV | "L" | 1.5 | 0.5 | 90 | 0 | 0.5 | 2.0 | - |
| V | "L" | 1.5 | 0.5 | 90 | 0 | 1.0 | 1.5 | - |
| VI | "L" | 1.35 | 0.4 | 90 | 0 | 0.8 | 1.2 | - |
| VII | "C" | 1.5 | 0.5 | 90 | 90 | 1.0 | 1.0 | 1.0 |
| VIII | "C" | 1.5 | 0.5 | 90 | 90 | 0.5 | 1.0 | 1.5 |
| IX | "C" | 1.35 | 0.4 | 90 | 90 | 0.8 | 0.8 | 0.8 |
| X | "C" | 1.35 | 0.4 | 90 | 90 | 0.4 | 0.8 | 1.2 |

Table 1B Parameters of exemplary implanted prostheses under clinical conditions

| Variant of the prosthesis | Cross section shape | d [mm] | P [mm] | $\alpha 1$ [°] | $\alpha 2$ [°] | Lm [mm] | Lk [mm] | Ls [mm] |
|---|---|---|---|---|---|---|---|---|
| A | "L" | 1.18 | 0.4 | 90 | 0 | 0.4 | 0.8 | - |
| B | "L" | 1.18 | 0.4 | 90 | 0 | 0.4 | 1.2 | - |
| C | "L" | 1.18 | 0.4 | 90 | 0 | 0.8 | 0.8 | - |
| D | "L" | 1.18 | 0.4 | 90 | 0 | 0.8 | 1.2 | - |
| E | "L" | 1.35 | 0.4 | 90 | 0 | 0.4 | 0.8 | - |
| F | "L" | 1.35 | 0.4 | 90 | 0 | 0.4 | 1.2 | - |
| G | "L" | 1.35 | 0.4 | 90 | 0 | 0.8 | 0.8 | - |
| H | "L" | 1.35 | 0.4 | 90 | 0 | 0.8 | 1.2 | - |
| I | "C" | 1.18 | 0.4 | 90 | 90 | 0.4 | 0.8 | 0.4 |
| J | "C" | 1.18 | 0.4 | 90 | 90 | 0.8 | 1.2 | 0.8 |
| K | "C" | 1.18 | 0.4 | 90 | 90 | 1.2 | 1.6 | 1.2 |
| L | "L" | 1.18 | 0.4 | 0 | 0 | 1.2 | 0 | - |
| M | "L" | 1.18 | 0.4 | 0 | 0 | 1.6 | 0 | - |
| N | "L" | 1.18 | 0.4 | 0 | 0 | 2.0 | 0 | - |
| O | "L" | 1.35 | 0.4 | 0 | 0 | 1.2 | 0 | - |
| P | "L" | 1.35 | 0.4 | 0 | 0 | 1.6 | 0 | - |
| R | "L" | 1.35 | 0.4 | 0 | 0 | 2.0 | 0 | - |
| S | "L" | 1.5 | 0.5 | 0 | 0 | 0.8 | 0 | - |
| T | "L" | 1.5 | 0.5 | 0 | 0 | 1.2 | 0 | - |

(continued)

| Variant of the prosthesis | Cross section shape | d [mm] | P [mm] | α1 [°] | α2 [°] | Lm [mm] | Lk [mm] | Ls [mm] |
|---|---|---|---|---|---|---|---|---|
| U | "L" | 1.6 | 0.5 | 0 | 0 | 0.8 | 0 | - |
| W | "L" | 1.6 | 0.5 | 0 | 0 | 1.2 | 0 | - |

Some of the variants of the middle ear prostheses 1 are shown in the axonometric projections in the figures: variant I - fig. 3A, variant II - fig. 3B, variant VI - fig. 3C, variant VII - fig. 4B and variant VIII - fig. 4A, respectively.

For the variants of the middle ear prosthesis presented in Table 1, the transmission of the acoustic wave, which can be described as a function of the spectral transmittance, i.e. the ratio of the input signal (acoustic wave) to the output signal as a frequency function, was determined according to the formula:

$$G(j\omega) = \frac{Y(j\omega)}{X(j\omega)}$$

For the safe and proper operation of the middle ear prosthesis 1, the function $G(j\omega)$ value for any frequency in the range 20 Hz ÷ 20 kHz should be in the range of ± 20 dB. This range corresponds to a 10-fold amplification or attenuation of the acoustic signal in a linear scale. Particular attention should be paid to high attenuation values (greater than -10 dB) for the frequency band from the range of audiometry tests, or 125 Hz ÷ 8 kHz. This fact is associated with good transmission of the acoustic wave in the range of speech signal occurrence. The stimulation in the study was a forcing sinusoidal variable pressure level of 80 dB (corresponding to an acoustic pressure equal to 0.2 Pa).

Table 2 presents the function $G(j\omega)$ value for the following variant of material parameters:

$$E = 11.38 \cdot 10^{10} \text{ N/m}^2,\ \nu=0.34,\ \rho = 4.43 \cdot 10^3 \text{ kg/m}^3.$$

Table 3 presents the function $G(j\omega)$ value for the following variant of material parameters:

$$E = 6.7 \cdot 10^{10} \text{ N/m}^2,\ \nu=0.33,\ \rho = 2.7 \cdot 10^3 \text{ kg/m}^3.$$

Analysis of the obtained results indicates that the amplitudes do not reach critical values in the low frequency range (20Hz - 400Hz). Therefore, the middle ear prosthesis 1 in the presented variants propagates well vibro-acoustic energy, without posing a threat of dangerous resonance in low frequencies.

**Example 3**

The middle ear prosthesis 1 according to the present invention was examined in simulation tests on middle ear and auditory ossicles models. Simulations were performed on temporal bone models using an operating microscope, a surgical drive together with its equipment and adequate surgical instruments, so as to simulate the intraoperative conditions of the reconstruction of the middle ear transmission system the most precisely possible. In the trials, pathologies of individual auditory ossicles were simulated. The results showed the following indications for the application of the proposed prosthesis: PORP-type reconstruction, TORP-type reconstruction, incudostapedial anastomosis, anvil interposition, aid in solving various difficult and unusual situations (e.g. "empty cavity").

Fig. 5 is a photograph of an implanted middle ear prosthesis 1 according to one embodiment of the present invention replacing a damaged anvil, whereas fig. 6 is a photograph of an implanted middle ear prosthesis 1 according to one embodiment of the present invention, stabilizing a disengaged incudostapedial joint. In the example presented in fig. 5 the middle ear prosthesis 1, and in fact the first spring element 2, was attached to the head of the stapes 5, while on the other side, the third spring element 4 was attached to the manubrium of the hammer 6. In this case, the middle ear prosthesis 1 served as an acoustic transducer ensuring the reconstruction of the middle ear conductive chain. On the other hand, in the example presented in fig. 5 the middle ear prosthesis 1 was attached between the head of the stapes 5 and the long crus of the anvil 7. In this case, the usefulness of the middle ear prosthesis 1 according to the present invention as an annular stabilizer of the incudostapedial joint was demonstrated.

The methodology for the use of middle ear prosthesis 1 according to the present invention consisted in the appropriate selection of the correct length and shape, changing the obtained length by cutting with a surgical knife or scissors of a relevant fragment of the produced element and/or changing the form made by the operator using microsurgical needles bending the prosthesis 1 to obtain a required shape.

**Example 4** Clinical tests

The prostheses according to the invention have been used in the surgical treatment of middle ear disorders requiring a reconstruction of the ossicular chain.

The results of observations of patients qualified for the clinical trial "UNISPRING - a new system of modelled microprostheses of auditory ossicles reconstructing the middle ear conductive chain".

In the group participating in the clinical trial, different variants of the prostheses according to the invention were implanted in 13 patients divided into two groups: 7 people and 6 people. The second group of patients has not completed the full observation period yet, but the results are so far encouraging. The results presented below originate from the analysis of data collected during the observation of patients from the first group.

| Type of surgery | Description | Prosthesis | Manufacture method |
|---|---|---|---|
| INCUDO-STAPEDOPEXIA | incudostapedial anastomosis | UNISPRING 1.18 UNISPRING 1.35 | L: short segment: 1 - 2 coils long segment: 2 - 3 coils |
| MALEO-STAPE-DOPEXIA | maleotapedial anastomosis | UNISPRING 1.18 UNISPRING 1.35 | C: 1. arm: 1 - 3 coils central arm: 2 - 4 coils 3. arm: 1 - 3 coils |
| PORP | partial ossicular replacement prosthesis | UNISPRING 1.50 UNISPRING 1.60 | straight segment of the length: 2 - 3 coils |
| TORP | total ossicular replacement prosthesis | UNISPRING 1.18 UNISPRING 1.35 | straight segment of the length: 3 - 5 coils |

The prosthesis according to the invention has proved to be a safe, effective, functional and ergonomic solution intended for the surgical treatment of the ossicular conductive chain.

The table below presents the scope of applications for which the prosthesis according to the invention was used, allowing for individual adjustment to the anatomical conditions of the patient and the type of pathology.

DESIGNATION OF PROSTHESIS TYPE:

| Prosthesis designation | Desciption |
|---|---|
| UNISPRING 1.18 | A cylindrical spring with an outer diameter of 1.18 mm, and length and shape adapted to anatomical conditions |
| UNISPRING 1.35 | A cylindrical spring with an outer diameter of 1.35 mm, and length and shape adapted to anatomical conditions |
| UNISPRING 1.50 | A cylindrical spring with an outer diameter of 1.50 mm, and length and shape adapted to anatomical conditions |
| UNISPRING 1.60 | A cylindrical spring with an outer diameter of 1.60 mm, and length and shape adapted to anatomical conditions |

Detailed evaluation of the effectiveness of the so far collected material for the whole I group has been made based on the analysis of the results of the pure tone audiometry, speech audiometry and a subjective assessment and it is presented below:

**A.:**

Pure tone audiometry - mean values of pre-operative reserve and improvement (reduction in the post-operative reserve by the following values):

| frequency [Hz] | The value of the auditory reserve before the procedure [dB] | Average improvement by: [dB]12 months after the procedure |
|---|---|---|
| 125 | 39 | 18 |
| 250 | 44 | 35 |
| 500 | 39 | 25 |
| 1000 | 27 | 20 |
| 2000 | 16 | 7 |
| 4000 | 15 | 15 |
| 8000 | 5 | 0 |

An improvement of the results within the described spectrum, including the so-called "closing reserve" phenomenon (value of the auditory reserve below 10 dB) for frequencies relevant to speech reception was obtained - for the following frequencies: 250 Hz, 500 Hz, 1 000 Hz, 2 000 Hz, 4 000 Hz.

**B.:**

Speech audiometry - mean values:

| loudness level [dB] | Understanding [%] | |
|---|---|---|
| | Before the procedure | 12 months after the procedure |
| 10 | 0 | 0 |
| 20 | 0 | 0 |
| 30 | 0 | 0 |
| 40 | 0 | 0 |
| 50 | 0 | 2 |
| 60 | 7 | 2 |
| 70 | 40 | 72 |
| 80 | 69 | 87 |
| 90 | 85 | 87 |
| 100 | 87 | 87 |

A significant improvement in the threshold of understanding was achieved - a reduction in the loudness level by 20 dB (from 60 to 40 dB). The maximum capacity of using the auditory capabilities (90-100% of test understanding) in the loudness range of 60 - 80 dB was achieved in all operated patients. Before the procedure, a similar situation occurred in 1 patient (100% understanding at 60 dB).

**C.:**

Average value of subjective assessment:

EP 3 600 154 B1

| Average value of subjective assessment [points] | |
| --- | --- |
| Before the procedure | 12 months after the procedure |
| 2.6 | 8.7 |

In all patients, an improvement of hearing assessed subjectively in the scale of 0- 10 points was achieved.

Table 2. Function of the spectral transmittance of the prosthesis for a material having the following parameters: $E = 11.38 \cdot 10^{10}$ N/m$^2$, v=0.34, $\rho = 4.43 \cdot 10^3$ kg/m$^3$.

| | f[Hz] | 20 | 25 | 32 | 40 | 50 | 63 | 80 | 100 | 125 | 160 | 200 | 250 | 315 | 400 | 500 | 630 | 800 | 1000 | 1250 | 1600 | 2000 | 2500 | 3000 | 3150 | 4000 | 5000 | 6300 | 8000 | 10000 | 12500 | 16000 | 20000 |
|---|---|---|---|---|---|---|---|---|---|---|---|---|---|---|---|---|---|---|---|---|---|---|---|---|---|---|---|---|---|---|---|---|---|
| | | | | | | | | | | | | | | | | | | | G[jω][dB] | | | | | | | | | | | | | | |
| Prosthesis variant | 1 | 10,1 | 10,1 | 10,1 | 10,1 | 10,1 | 10,1 | 10,1 | 10,1 | 10,1 | 10,1 | 10,1 | 10,1 | 10,1 | 10,1 | 0,0 | 0,0 | 0,0 | 0,1 | 0,2 | 0,4 | 0,7 | 1,1 | 1,7 | 1,9 | 3,3 | 5,7 | 9,7 | 8,0 | 1,7 | 12,5 | 13,8 | 3,2 |
| | 2 | 0,5 | 0,5 | 0,5 | 0,5 | 0,5 | 0,5 | 0,5 | 0,5 | 0,5 | 0,5 | 0,5 | 0,5 | 0,6 | 0,6 | 0,6 | 0,7 | 0,9 | 1,1 | 1,4 | 2,0 | 3,0 | 4,6 | 6,7 | 7,7 | 12,4 | 8,2 | 2,4 | 10,4 | 10,7 | 4,9 | 10,1 | 5,1 |
| | 3 | 10,1 | 10,1 | 10,1 | 10,1 | 10,1 | 10,1 | 10,1 | 10,1 | 10,1 | 10,1 | 10,1 | 10,1 | 10,1 | 10,1 | 0,0 | 0,0 | 0,1 | 0,2 | 0,4 | 0,7 | 1,2 | 2,0 | 3,0 | 3,4 | 5,9 | 9,8 | 7,2 | 1,1 | 12,9 | 14,2 | 10,7 | 4,7 |
| | 4 | 11,5 | 11,5 | 11,5 | 11,5 | 11,5 | 11,5 | 11,5 | 11,5 | 11,5 | 11,5 | 11,5 | 11,4 | 11,4 | 11,3 | 11,2 | 11,0 | 10,7 | 10,2 | 0,6 | 1,9 | 3,8 | 5,8 | 4,8 | 4,1 | 0,7 | 11,2 | 13,8 | 14,4 | 4,5 | 11,6 | 2,9 | 6,5 |
| | 5 | 12,2 | 12,2 | 12,2 | 12,2 | 12,2 | 12,2 | 12,2 | 12,2 | 12,2 | 12,2 | 12,1 | 12,1 | 12,1 | 12,1 | 12,0 | 11,9 | 11,7 | 11,4 | 10,9 | 10,1 | 1,1 | 3,1 | 4,6 | 4,7 | 3,1 | 10,3 | 12,0 | 11,2 | 1,4 | 2,4 | 0,6 | 5,4 |
| | 6 | 11,6 | 11,6 | 11,6 | 11,6 | 11,6 | 11,6 | 11,6 | 11,6 | 11,6 | 11,6 | 11,5 | 11,5 | 11,5 | 11,5 | 11,4 | 11,4 | 11,3 | 11,1 | 10,8 | 10,3 | 0,4 | 1,6 | 3,0 | 3,6 | 5,9 | 4,5 | 0,6 | 12,1 | 12,5 | 3,1 | 1,5 | 6,0 |
| | 7 | 16,5 | 16,5 | 16,5 | 16,5 | 16,5 | 16,5 | 16,5 | 16,5 | 16,5 | 16,5 | 16,5 | 16,5 | 16,5 | 16,4 | 16,4 | 16,3 | 16,2 | 16,0 | 15,7 | 15,2 | 14,3 | 13,1 | 12,1 | 12,0 | 5,9 | 1,8 | 1,2 | 10,4 | 10,0 | 15,9 | 3,2 | 7,1 |
| | 8 | 16,3 | 16,3 | 16,3 | 16,3 | 16,3 | 16,3 | 16,3 | 16,3 | 16,3 | 16,3 | 16,3 | 16,2 | 16,2 | 16,2 | 16,1 | 16,0 | 15,8 | 15,5 | 15,0 | 14,3 | 13,2 | 11,9 | 11,7 | 11,4 | 3,1 | 4,1 | 12,6 | 14,8 | 13,6 | 4,7 | 13,5 | 3,3 |
| | 9 | 17,3 | 17,3 | 17,3 | 17,3 | 17,3 | 17,3 | 17,3 | 17,3 | 17,3 | 17,3 | 17,3 | 17,3 | 17,3 | 17,2 | 17,2 | 17,1 | 17,0 | 16,9 | 16,7 | 16,3 | 15,7 | 14,6 | 13,4 | 12,9 | 0,1 | 1,6 | 1,0 | 11,5 | 10,9 | 11,1 | 11,5 | 4,2 |
| | 10 | 18,9 | 18,9 | 18,9 | 18,9 | 18,9 | 18,9 | 18,9 | 18,9 | 18,9 | 18,9 | 18,9 | 18,9 | 18,8 | 18,8 | 18,8 | 18,7 | 15,6 | 18,4 | 18,1 | 17,6 | 16,9 | 15,6 | 14,4 | 14,0 | 12,5 | 3,1 | 0,5 | 12,5 | 14,1 | 0,8 | 1,7 | 7,4 |

Table 3. Function of the spectral transmittance of the prosthesis for a material having the following parameters: $E = 6.7 \cdot 10^{10}$ N/m$^2$, $v= 0.33$, $\rho = 2.7 \cdot 10^3$ kg/m$^3$.

| | f[Hz] | 20 | 25 | 32 | 40 | 50 | 63 | 80 | 100 | 125 | 160 | 200 | 250 | 315 | 400 | 500 | 630 | 800 | 1000 | 1250 | 1600 | 2000 | 2500 | 3000 | 3150 | 4000 | 5000 | 6300 | 8000 | 10000 | 12500 | 16000 | 20000 |
|---|---|---|---|---|---|---|---|---|---|---|---|---|---|---|---|---|---|---|---|---|---|---|---|---|---|---|---|---|---|---|---|---|---|
| Prosthesis variant | 1 | 10,1 | 10,1 | 10,1 | 10,1 | 10,1 | 10,1 | 10,1 | 10,1 | 10,1 | 10,1 | 10,1 | 10,1 | 10,1 | 10,1 | 0,0 | 0,0 | 0,0 | 0,1 | 0,2 | 0,4 | 0,7 | 1,2 | 1,7 | 2,0 | 3,4 | 5,9 | 10,0 | 7,6 | 7,5 | 4,8 | 0,7 | 5,5 |
| | 2 | 0,5 | 0,5 | 0,5 | 0,5 | 0,5 | 0,5 | 0,5 | 0,5 | 0,5 | 0,5 | 0,5 | 0,5 | 0,6 | 0,6 | 0,6 | 0,7 | 0,9 | 1,1 | 1,5 | 2,0 | 3,0 | 4,8 | 7,0 | 8,0 | 12,5 | 7,8 | 2,2 | 10,5 | 11,3 | 6,6 | 10,6 | 4,8 |
| | 3 | 10,1 | 10,1 | 10,1 | 10,1 | 10,1 | 10,1 | 10,1 | 10,1 | 10,1 | 10,1 | 10,1 | 10,1 | 10,1 | 10,1 | 0,0 | 0,0 | 0,1 | 0,2 | 0,4 | 0,7 | 1,2 | 2,1 | 3,1 | 3,5 | 6,1 | 9,9 | 6,8 | 0,8 | 13,1 | 14,1 | 11,0 | 4,6 |
| | 4 | 11,5 | 11,5 | 11,5 | 11,5 | 11,5 | 11,5 | 11,5 | 11,5 | 11,5 | 11,5 | 11,5 | 11,4 | 11,4 | 11,3 | 11,2 | 11,0 | 10,7 | 10,2 | 0,6 | 2,0 | 4,0 | 5,8 | 4,6 | 3,9 | 0,5 | 11,2 | 12,2 | 15,1 | 3,8 | 12,4 | 11,1 | 4,5 |
| | 5 | 12,2 | 12,2 | 12,2 | 12,2 | 12,2 | 12,2 | 12,2 | 12,2 | 12,2 | 12,2 | 12,1 | 12,1 | 12,1 | 12,0 | 12,0 | 11,9 | 11,7 | 11,4 | 10,9 | 0,0 | 1,2 | 3,2 | 4,6 | 4,7 | 2,9 | 10,5 | 12,0 | 1,9 | 0,4 | 2,6 | 10,6 | 4,8 |
| | 6 | 11,6 | 11,6 | 11,6 | 11,6 | 11,6 | 11,6 | 11,6 | 11,6 | 11,6 | 11,6 | 11,5 | 11,5 | 11,5 | 11,5 | 11,4 | 11,4 | 11,3 | 11,1 | 10,8 | 10,3 | 0,4 | 1,7 | 3,1 | 3,7 | 5,9 | 4,3 | 0,4 | 12,1 | 12,5 | 2,5 | 1,5 | 6,0 |
| | 7 | 16,5 | 16,5 | 16,5 | 16,5 | 16,5 | 16,5 | 16,5 | 16,5 | 16,5 | 16,5 | 16,5 | 16,5 | 16,5 | 16,4 | 16,4 | 16,3 | 16,2 | 16,0 | 15,6 | 15,1 | 14,3 | 13,0 | 12,0 | 12,1 | 5,5 | 1,5 | 2,0 | 0,0 | 7,4 | 15,9 | 10,3 | 4,9 |
| | 8 | 16,3 | 16,3 | 16,3 | 16,3 | 16,3 | 16,3 | 16,3 | 16,3 | 16,3 | 16,3 | 16,3 | 16,2 | 16,2 | 16,2 | 16,1 | 16,0 | 15,8 | 15,5 | 15,0 | 14,2 | 13,1 | 11,9 | 11,6 | 11,3 | 3,8 | 2,2 | 13,0 | 14,7 | 11,6 | 4,8 | 10,8 | 4,7 |
| | 9 | 17,3 | 17,3 | 17,3 | 17,3 | 17,3 | 17,3 | 17,3 | 17,3 | 17,3 | 17,3 | 17,3 | 17,3 | 17,3 | 17,2 | 17,1 | 17,0 | 16,9 | 16,6 | 16,3 | 15,6 | 14,6 | 13,3 | 12,8 | 0,4 | 1,8 | 0,9 | 12,8 | 10,5 | 8,7 | 11,3 | 4,4 | |
| | 10 | 18,9 | 18,9 | 18,9 | 18,9 | 18,9 | 18,9 | 18,9 | 18,9 | 18,9 | 18,9 | 18,9 | 18,9 | 18,8 | 18,8 | 18,8 | 18,7 | 18,6 | 18,4 | 18,1 | 17,6 | 16,8 | 15,5 | 14,3 | 13,8 | 12,3 | 3,8 | 10,7 | 12,6 | 13,9 | 10,2 | 3,0 | 6,9 |

## EP 3 600 154 B1

**Claims**

1. A middle ear prosthesis (1) of the middle ear conductive chain, **characterized in that** it consists of:

   - a first spring element (2) constituting a spring of at least one coil, having a length $L_m$ in the range from 0.2 mm to 10 mm,
   and possibly
   - a second spring element (3) constituting a spring of at least one coil, having a length $L_k$ in the range from 0.1 mm to 4 mm, wherein the spring elements (2, 3) form a continuous structure and the first spring element (2) is connected to the second spring element (3) in such a way that the rotational symmetry axis of the first spring element (2) is placed at an angle $\alpha_1$ with respect to the rotational symmetry axis of the second spring element (3), included in the range from 5° to 160°,
   and possibly
   - a third spring element (4) attached to the second spring element (3) at the end opposite to the first spring element (2), wherein the third spring element (4) is a spring of at least one coil, having a length $L_s$ in the range from 0.1 mm to 1 mm, wherein the spring elements (2, 3, 4) form a continuous structure and the third spring element (4) is connected to the second spring element (3) in such a way that the rotational symmetry axis of the third spring element (4) is placed at an angle $\alpha_2$ with respect to the rotational symmetry axis of the second spring element (3), included in the range from 5° to 160°,

   wherein the whole middle ear prosthesis (1) is made of wire of a material having a Young's modulus E in the range from $7 \cdot 10^{10}$ N/m² to $11.4 \cdot 10^{10}$ N/m², a density $\rho$ in the range from $4 \cdot 10^3$ kg/m³ to $20 \cdot 10^3$ kg/m³, a Poisson's ratio v in the range from 0.34 to 0.44,

2. The middle ear prosthesis (1) according to claim 1, wherein the spring elements (2, 3, 4) form a compression cylindrical spring, preferably with an outer diameter $D_z$ in the range from 1.0 mm to 1.6 mm.

3. The middle ear prosthesis (1) according to any of the claims from 1 to 2, wherein the spring elements (2, 3, 4) are made of wire of a circular cross section having a diameter d in the range from 0.1 mm to 0.3 mm.

4. The middle ear prosthesis (1) according to any of the claims from 1 to 3, wherein the spring elements (2, 3, 4) have a spring pitch P in the range from 0.1 mm to 1.0 mm.


**Patentansprüche**

1. Mittelohrprothese (1) der Mittelohrleitungskette, **dadurch gekennzeichnet, dass** sie besteht aus:

   - einem ersten Federelement (2), das eine Feder aus mindestens einer Windung bildet und eine Länge $L_m$ in dem Bereich von 0,2 mm bis 10 mm aufweist,
   und gegebenenfalls
   - einem zweiten Federelement (3), das eine Feder aus mindestens einer Windung bildet und eine Länge $L_k$ in dem Bereich von 0,1 mm bis 4 mm aufweist, wobei die Federelemente (2, 3) eine durchgehende Struktur bilden und das erste Federelement (2) mit dem zweiten Federelement (3) derart verbunden ist, dass die Rotationssymmetrieachse des ersten Federelements (2) in einem Winkel $\alpha_1$ in Bezug auf die Rotationssymmetrieachse des zweiten Federelements (3) platziert ist, der in dem Bereich von 5° bis 160° liegt,
   und gegebenenfalls
   - einem dritten Federelement (4), das an dem Ende gegenüber dem ersten Federelement (2) an dem zweiten Federelement (3) befestigt ist, wobei das dritte Federelement (4) eine Feder aus mindestens einer Windung ist, die eine Länge $L_s$ in dem Bereich von 0,1 mm bis 1 mm aufweist, wobei die Federelemente (2, 3, 4) eine durchgehende Struktur bilden und das dritte Federelement (4) mit dem zweiten Federelement (3) derart verbunden ist, dass die Rotationssymmetrieachse des dritten Federelements (4) in einem Winkel $\alpha_2$ in Bezug auf die Rotationssymmetrieachse des zweiten Federelements (3) platziert ist, der in dem Bereich von 5° bis 160° liegt,

   wobei die gesamte Mittelohrprothese (1) aus einem Draht aus einem Material, das einen Elastizitätsmodul E in dem Bereich von $7 \cdot 10^{10}$ N/m² bis $11,4 \cdot 10^{10}$ N/m², eine Dichte p in dem Bereich von $4 \cdot 10^3$ kg/m³ bis $20 \cdot 10^3$ kg/m³ und eine Poissonzahl V in dem Bereich von 0,34 bis 0,44 aufweist, gefertigt ist.

**2.** Mittelohrprothese (1) nach Anspruch 1, wobei die Federelemente (2, 3, 4) eine Druckzylinderfeder bilden, bevorzugt mit einem Außendurchmesser $D_z$ in dem Bereich von 1,0 mm bis 1,6 mm.

**3.** Mittelohrprothese (1) nach einem der Ansprüche 1 bis 2, wobei die Federelemente (2, 3, 4) aus Draht mit einem kreisförmigem Querschnitt, der einen Durchmesser d in dem Bereich von 0,1 mm bis 0,3 mm aufweist, gefertigt sind.

**4.** Mittelohrprothese (1) nach einem der Ansprüche 1 bis 3, wobei die Federelemente (2, 3, 4) eine Federsteigung P in dem Bereich von 0,1 mm bis 1,0 mm aufweisen.

**Revendications**

**1.** Prothèse d'oreille moyenne (1) de la chaîne conductrice d'oreille moyenne, **caractérisée en ce qu'elle** consiste en :

- un premier élément ressort (2) constituant un ressort d'au moins une spire, présentant une longueur $L_m$ comprise dans la plage de 0,2 mm à 10 mm,
et éventuellement
- un deuxième élément ressort (3) constituant un ressort d'au moins une spire, présentant une longueur $L_k$ comprise dans la plage de 0,1 mm à 4 mm, dans laquelle les éléments ressorts (2, 3) forment une structure continue et le premier élément ressort (2) est relié au deuxième élément ressort (3) de telle sorte que l'axe de symétrie de rotation du premier élément ressort (2) soit placé selon un angle $\alpha_1$ par rapport à l'axe de symétrie de rotation du deuxième élément ressort (3) inclus dans la plage de 5° à 160°,
et éventuellement
- un troisième élément ressort (4) fixé au deuxième élément ressort (3) à l'extrémité opposée du premier élément ressort (2), dans laquelle le troisième élément ressort (4) est un ressort d'au moins une spire, présentant une longueur $L_s$ comprise dans la plage de 0,1 mm à 1 mm, dans laquelle les éléments ressorts (2, 3, 4) forment une structure continue et le troisième élément ressort (4) est relié au deuxième élément ressort (3) de telle sorte que l'axe de symétrie de rotation du troisième élément ressort (4) soit placé selon un angle $\alpha_2$ par rapport à l'axe de symétrie de rotation du deuxième élément ressort (3) inclus dans la plage de 5° à 160°,

dans laquelle l'intégralité de la prothèse d'oreille moyenne (1) est composée de fils d'un matériau présentant un Module E de Young compris dans la plage de $7 \cdot 10^{10}$ N/m² à $11,4 \cdot 10^{10}$ N/m², une densité p comprise dans la plage de $4 \cdot 10^3$ kg/m³ à $20 \cdot 10^3$ kg/m³, un coefficient V de Poisson compris dans la plage de 0,34 à 0,44.

**2.** Prothèse d'oreille moyenne (1) selon la revendication 1, dans laquelle les éléments ressorts (2, 3, 4) forment un ressort cylindrique de compression, de préférence avec un diamètre extérieur $D_z$ compris dans la plage de 1,0 mm à 1,6 mm.

**3.** Prothèse d'oreille moyenne (1) selon l'une quelconque des revendications 1 à 2, dans laquelle les éléments ressorts (2, 3, 4) sont composés de fils d'une section transversale circulaire présentant un diamètre d compris dans la plage de 0,1 mm à 0,3 mm.

**4.** Prothèse d'oreille moyenne (1) selon l'une quelconque des revendications 1 à 3, dans laquelle les éléments ressorts (2, 3, 4) présentent un pas de ressort P compris dans la plage de 0,1 mm à 1,0 mm.

**Fig. 1**

**Fig. 2**

A)                          B)                          C)

**Fig. 3**

2

3

4

A)

2

3

4

B)

**Fig. 4**

6

5

1

**Fig. 5**

1

5

7

**Fig. 6**

# EP 3 600 154 B1

## REFERENCES CITED IN THE DESCRIPTION

### Patent documents cited in the description

- PL 217562 B1
- US 6277148 B1